# NEUE EUROPÄISCHE PATENTSCHRIFT

(11) **EP 0 854 726 B2**
(45) Veröffentlichungstag und Bekanntmachung des Hinweises auf die Entscheidung über den Einspruch: **29.07.2009**
(45) Hinweis auf die Patenterteilung: 16.12.1998
(21) Anmeldenummer: 96945476.8
(22) Anmeldetag: 27.09.1996
(51) Int. Cl.: A61K 36/38

(54) **STABILER EXTRAKT AUS HYPERICUM PERFORATUM L., VERFAHREN ZU SEINER HERSTELLUNG UND PHARMAZEUTISCHE ZUBEREITUNGEN**
STABLE EXTRACT OF HYPERICUM PERFORATUM L., PROCESS FOR PREPARING THE SAME AND PHARMACEUTICAL COMPOSITIONS
EXTRAIT STABLE D'HYPERICUM PERFORATUM L., SON PROCEDE DE PREPARATION ET COMPOSITIONS PHARMACEUTIQUES

(30) Priorität: 29.09.1995 DE 19536496; 22.03.1996 DE 19611374; 14.05.1996 DE 19619512
(43) Veröffentlichungstag der Anmeldung: 29.07.1998
(73) Patentinhaber: Dr. Willmar Schwabe GmbH & Co. KG, 76227 Karlsruhe (DE)
(72) Erfinder: ERDELMEIER, Clemens, D-76139 Karlsruhe (DE); GRETHLEIN, Eckhart, D-76327 Pfinztal (DE); LANG, Friedrich, D-76767 Hagenbach (DE); OSCHMANN, Rainer, D-76829 Landau (DE)
(74) Vertreter: Adam, Holger
(86) Internationale Anmeldenummer: PCT/DE1996/001876
(87) Internationale Veröffentlichungsnummer: WO 1997/013489

(56) Entgegenhaltungen:
- EP-A- 0 599 307
- GB-A- 2 101 888
- RO-A- 79 428
- DATABASE WPI Section Ch, Week 8337 Derwent Publications Ltd., London, GB; Class B05, AN 83-762732 XP002033079 & RO 79 428 A (INTR MED COLORANTI SINTOFARM) , 28.Februar 1983
- THERAPIEWOCHE, Bd. 44, Nr. 14, April 1994, Seiten 808-815, XP000674704 STEFFI SATTLER ET AL.: "JOHANNISKRAUT.EIN REZEPT DER NATUR GEGEN DEPRESSIONEN."
- R. HÄNSEL - K. KELLER - H. RIMPLER - G. SCHNEIDER: 'Drogen E-O', Bd. 5, 1993, SPRINGER-VERLAG, BERLIN HEIDELBERG NEWYORK Seiten 474 - 495
- Dissertation vorgelegt von Peter Maisenbacher (1991) "Untersuchungen zur Analytik von Johanniskrautöl" (auszugsweise)

## Beschreibung

Durch pharmalaologische und klinische. Versuche ist belegt, daß Extrakte aus Johanniskraut (Hypericumextrakte) bei depressiven Verstimmungszuständen bis hin zu mittelschweren Depressionen mit Erfolg eingesetzt werden können. Die milde antidepressive Gesamtwirkung konnte jedoch noch nicht eindeutig einem oder mehreren Inhaltsstoffen zugeordnet werden; vgl. J. Hötzl, S. Sattler und H. Schütt, Johanniskraut: eine Alternative zu synthetischen Antidepressiva, Pharmazeutische Zeitung Nr. 46, 139. Jahrgang, 17. November 1994, 3959-3977. Allerdings gibt es gerade in neuester Zeit verstärkt Hinweise, daß zur Erzielung der Wirksamkeit Hyperforin wesentlich beiträgt (EP-A-0 599 307).

Die Arzneidroge besteht aus den oberirdischen Teilen von Hypericum perforatum L Die Inhaltsstoffe von Hypericum perforatum L. sind unter anderem Hypericin und Hyperforin; vgl. J. Hölzl et al., a.a.O.

Die Herstellung von Hypericumextrakten mit angereichertem Hypericin-Gehalt ist in der DE-PS 1 569 849 sowie von S. Niesel und H. Schilcher in Arch. Pharm., Bd. 323 (1990), 755 beschrieben.

Aus der Arbeit von R. Berghöfer und J. Hölzl, Deutsche Apothekerzeitung, Bd. 126, Nr. 47 (1986), Seiten 2569. 2573, ist bekannt, daß Hyperforin in Extrakten aus gelagerter Droge bereits nach einer Woche völlig abgebaut ist, während es im Frischpflanzenextrakt stabiler sein soll. Die Verfasser vermuten, daß Frischpflanzen einen Stabilisator für Hyperforin enthalten. J. Hötzl et al., Planta Med., Bd. 55 (1989), Seiten 601 - 602, berichten über Hypericumöl und einen vermuteten Zusammenhang zwischen der Hypericinkonzentration und der Peroxidzahl) (POZ). Dem Sonnenlicht ausgesetzte Hypericumötpraparate zeigen unterschiedliche Peroxidzahlen. Nach J. Hölzl et al. besteht aber kein Zusammenhang zwischen dem POZ-Wert und der Hypericinlaonzentration.

Über die Stabilität von Hypericumöl berichten P. Maisenbacher und K.-A. Kovar in Planta Med., Bd. 58 (1992), Seiten 351 - 354. Dieses Öl enthielt auch Hyperforin, das innerhalb weniger Wochen zersetzt war.

Aus der EP-A-0 599 307 (entspricht DE-OS 4 239 959) sind Hypericumextrakte und Verfahren zu ihrer Herstellung bekannt, die möglichst wenig Hypericin oder ähnliche photosensibilisierenden Verbindungen enthalten, aber dennoch die bisher dem Hypericin zugeschriebene Wirksamkeit besitzen. Diese kann auf das Vorhandensein von Hyperforin zurückgeführt werden.

Ferner ist bekannt, Hypericumöl (Johannisöl, Oleum hyperici) durch Extraktion von zerquetscMen frischen Johanniskrautblüten mit einem fetten Öl wie Olivenöl, Sojaöl, Weizenkeimlingsöl oder Sonnenblumenöl herzustellen. Hypericumöl enthält variable Mengen an Hyperforin und eignet sich zur äußerlichen Behandlung von Wunden, insbesondere Brandwunden und Verschürfungen; vgl. P. Maisenbacher und K.-A. Kovar, Planfa Med, Bd. 58 (1992), 351 - 354 und J. Hölzl, L Demisch und S. Stock, Planta Med, Bd. 55 (1989), 601 -602.

Sowohl in der Droge als auch in üblichen Hypericumextrakten nimmt der Hyperforingehalt bei normaler Lagerung innerhalb weniger Monate drastisch ab bis zum Verschwinden der Substanz; vgl. Dissertation von P. Maisenbacher, Tübingen 1991, und Dissertation von R. Berghöfer. Marburg/L 1987. In früheren Versuchen mit öligen Hypericumextrakten konnte die Stabilität hyperforinhaltiger Zubereitungen lediglich durch Lagerung unter Argon deutlich verbessert werden; vgl. Dissertation von P Maisenbacher, Tübingen 1991. Eine Stabilisierung durch Antioxidantien wie Butylhydroxytoluol) (BHT) und Butylhydroxyanisol (BHA) gelang in diesen Extrakten nicht. Desgleichen bringen übliche Antioxidantien wie Oxynex LM und Oxynex 2004 keine Verbesserung der Stabilität. Bei Hypericumöl wird nach P. Maisenbacher, Dissertation, loc. cit., die beste Stabilität durch Verwendung von Octyldodecanol (Eutanol G) als Extraktionsmittel erreicht; vgl. Dissertation P Maisenbacher 1991, Seiten 151-154.

Hyperforinhattige Hypericumextrakte können mit in der Pharmazie üblichen anorganischen oder organischen Lösungsmitteln oder deren Gemischen hergestellt werden (P. List und P.C. Schmidt, Technologie pflanzlicher Arzneizubereitungen, Wissensch. Verlagsgesellschaft mbH, Stuttgart 1984)

In üblichen ethanolisch-wässrigen Hypericumextrakten und daraus hergestellten Fertigarzneimitteln ist, bezogen auf den Extrakt, in der Regel weniger als ca. 1% Hyperforin enthalten. Nach Lagerung sinkt der Wert deutlich ab und geht je nach Lagerungsbedingungen gegen null. Man vermutet, daß Oxidationsprozesse für den Abbau des Hyperforins in der Droge und im Extrakt verantwortlich sind.

Der Erfindung liegt die Aufgabe zugrunde, verbesserte, Hyperforin enthaltende, stabilisierte Extrakte aus Hypericum perforatum L (Johanniskraut) bereitzustellen, in denen das Hyperforin über lange Zeit stabil bleibt. Eine weitere Aufgabe der Erfindung ist es, Verfahren zur Herstellung dieser stabilisierten Extrakte sowie diese enthaltende Arzneimittel bereitzustellen, in denen der Hyperforingehalt ebenfalls stabil bleibt.

Diese Aufgaben werden erfindungsgemäß durch die Extrakte gemäß den Patentansprüchen 1 bis 6, das Verfahren gemäß den Patentansprüchen 7 bis 17, sowie die pharmazeutische Zubereitung gemäß Patentanspruch 18 gelöst.

Die vorliegende Erfindung beruht unter anderem auf dem überraschenden Befund, daß durch bestimmte antioxidative und/oder sauerstoffbindende Stabilisatoren bzw. Reduktionsmittel, die in der Lage sind, Oxidantien wie z.B. Radikale, Peroxide, Luftsauerstoff etc. im Extrakt abzubauen und/oder den Abbau von Hyperforin zu hemmen, und gegebenenfalls Durchführung der Extraktion unter einem Inertgas wie Stickstoff und/oder Lichtausschluß ünd/oder eines in seinem Gehalt an freiem Sauerstoff stark verminderten Lösungsmittels, der so erhaltene Extrakt gegenüber einem unbehandelten Hypericumextrakt wesentlich länger stabil bleibt. Dieser Extrakt kann im Gegensatz zu den Beobachtungen von R Berghöfer und J. Hölzl, loc. cit. auch aus einer getrockneten, gelagerten Droge stammen.

Ein in seinem Sauerstoffgehalt stark vermindertes Lösungsmittel kann durch physikalische Behandlung. z.B. Spülen mit einem Inertgas wie Stickstoff, hergestellt werden. Wird ein Hypericumextrakt erfindungsgemäß konserviert bzw. stabilisiert, durch Zusatz eines Antioxidans aus der um Anspruch 1 angegeben Gruppe und vorzugsweise unter Ausschluß von Licht und Luftsauerstoff hergestellt, bleibt das Hyperforin in diesem Extrakt lange Zeit praktisch stabil. Der Schutz vor Licht und Luftsauerstoff kann auch durch eine entsprechende pharmazeutische Formulierung erreicht werden.

In einer bevorzugten Ausführungsform des erfindungsgemäßen Verfahrens zur Herstellung des stabilisierten Extraktes wird die frische oder vorzugsweise getrocknete Johanniskrautdroge mit wäßrigem Methanol oder Ethanol extrahiert, dessen Sauerstoffgehalt durch physikalische Behandlung stark vermindert wurde. Der Extraktlösung wird entsprechend gegebenenfalls vorliegenden Oxidantien, ein Antioxidationsmittel als Stabilisator zugesetzt und darin gelöst. Weitere Beispiele für bevorzugte Lösungsmittel zur Extraktion von Johanniskraut, umfassen die Gruppe der niedrig siedenden Alkane mit etwa 5 bis 8 C-Atomen. z.B. Pentane, Hexane und Heptane, insbesondere n-Heptan, und flüssiges oder überkritisches Kohlendioxid. Der Ausdruck wäßriges Methanol oder Ethanol bedeutet Methanol oder Ethanol mit einem Wassergehalt von vorzugsweise bis zu etwa 40 Vo).-%.

Antioxidative Stabilisatoren bzw. Antioxidationsmittel sind pharmalcologisch verträgliche Substanzen, die In der Lage sind, den Abbau von Hyperforin zu hemmen und/oder im Extrakt oder Arzneimittel enthaltende Oxidantien zu reduzieren, es sind dies Substanzen aus der Gruppe bestehend aus organischen Thiolverbindurigen, vorzugsweise Cystein und Glutathion, sowie Ascorbinsäure und Derivate dieser Verbindungen, wie die Fettsäureester der Ascorbinsäure, z.B. das Myristat, Palmitat und Stearat.

Diese antioxidativen Stabilisatoren werden in einer zur Stabilisierung des Hyperforins ausreichenden Menge der Hypericum-Extraktlösung einverleibt, wobei der antioxidative Stabilisator in einer Konzentration von 0,2 bis 5% bezogen auf den Hypericumexrtrakt, vorliegt.

in einer anderen Ausführungsform wird ebenfalls wie im Patentanspruch 7 verfahren, und der Zusatz des Stabilisators nach der Trocknung der Extraktlösung, d.h. nach dem Entfernen des Lösungsmittels ausgeführt.

Vorzugsweise werden alle Ausführungsformen unter Licht- und SauerstoffausschluB durchgeführt.

Die erhaltenen Extrakte können zusammen mit üblichen pharmazeutischen Hilfsstoffen, gegebenenfalls nach erneutem Zusatz eines Stabilisators, zu pharmazeutischen Zubereitungen wie Kapseln. Filmtabletten und Dragees verarbeitet werden.

Als pharmazeutische Hilfsstoffe werden übliche Füll-, Binde-, Spreng-, Schmier- und Überzugsmittel für Filmtabletten und Dragees sowie Öle und Fette als Füllmassen für WeichgeleMnakapsein verwendet.

Die Erfindung wird anhand folgender nicht einschränkender Beispiele weiter erläutert. Prozentangaben beziehen sich auf das Gewicht, sofern nichts anderes angegeben ist. Als Inertgas (Schutzgas) wurde Stickstoff verwendet. Es kann jedoch auch ein anderes Inertgas wie Argon oder Krypton verwendet werden.

### Beispiele 1 a) und 1b) (Vergleichsbeispiele).

a) 1 kg Johanniskrautdroge wurde in einer Mühle fein gemahlen und mit 7 kg 70(v/v)%-igem Ethanol versetzt. Die Suspension aus 1 kg Droge und 7 kg Lösungsmittel wurde eine Stunde bei 55°C unter Inertgas intensiv gerührt. Sodann wurde der erhaltene Extrakt von der Droge mittels einer Zentrifuge getrennt. Der Drogenrockstand wurde ein zweites Mal in gleicher Weise mit 7 kg Lösungsmittel extrahiert Die beiden Extrakflösungen wurden veréinigt, und mit einem Aliquot wurde der Trockenrückstand im Extrakt bestimmt. Der Extrakt wurde schonend unter vermindertem Druck auf einen Trackenrückstand von etwa 70 % konzentriert und bei 40°C unter vermindertem Druck nachgetrocknet. Es wurden 0,42 kg Trockenextrakt erhaben. Der Gehalt an Hyperforin betrug 2,26 %, der Gehalt an Gesamthypericinen 0,27 %.
b) Dieser Trockenextrakt aus Beispiel 1a) wurde mittels Beharidlung mit Polyvinylpyrrolidon (PVP) gemäß der Lehre der EP-A-O 599 307 von Hypericinen befreit Der Hyperforingehalt betrug 2,96%.

### Beispiel 2

24 kg Johanniskrautdroge wurden in einer Mühle fein gemahlen und mit 156 kg 80(v/v)%-igem Methanol versetzt, das vorher mit Stickstoff durchspült worden war. Dieses Gemisch wurde anschließend eine Stunde bei 55°C gerührt. Die erhaltene Extraktlösung wurde durch Zentrifugieren vom Drogenrückstand getrennt. Der Rückstand wurde nochmals in der gleichen Weise extrahiert Beide Extraktlösungen wurden vereinigt und mit 1,0 Gew-% Ascorbinsäure versetzt. Diese Lösung wurde während 15 Minuten geführt Dann wurde die Extraktlösung schonend unter vermindertem Druck eingeengt auf einen Trockenrückstandsgehaft von 70 %. Danach wurde bei 40 °C unter vermindertem Druck nachgetrocknet. Es resultierten 5,39 kg stabilisierter Trockenextrakt mit einem Gehalt an Hyperforin von 3,2 %. Der Gesamthypericingehalt in diesem Extrakt betrug 0,48 %.

### Beispiel 3

454 g fein geschnittenes frisches Johanniskraut wurden in einer Drogenpresse ausgepreßt. Dem Preßsaft (160 ml) wurden 1,5 g Ascorbinsäure zugesetzt und aufgelöst. Der Preßsaft wurde anschließend der ausgepreßten Droge wieder zugemischt. Dann wurden der feuchten Droge 1 kg n-Heptan zugegeben. Das Gemisch wurde während 1 Stunde unter ständigem Rühren bei 50 °C unter Lichtausschluß extrahiert. Anschließend wurde über ein Seitz Supra 1500 Filter abgesaugt und der Drogenrückstand noch ein zweites Mal in der gleichen Weise extrahiert. Die vereinigten Extraktlösungen wurden am Rotationsverdampfer bei 35°C unter lichtschutz auf einen Trockenrückstandsgehalt von ca. 70 % konzentriert und schließlich gefriergetrocknet. Es resultierte 9,11 g Trockenextrakt mit einem Gehalt an Hyperforin von 37,2 %.

### Beispiel 4

515 g fein geschnittenes frisches Johanniskraut wurden in einer Drogenpresse ausgepreßt. Dem Preßsaft (180 ml) wurden 1,7 g Ascorbinsäure zugesetzt und aufgelöst. Der Preßsaft wurde anschließend der ausgepreßten Droge wieder zugemischt. Dann wurde die feuchte Droge in eine Hochdruckextraktionsanlage gegeben und bei 350 bar bei 40 °C mit Kohlendioxid extrahiert. Pro kg Droge wurden 20 kg Kohlendioxid eingesetzt. Nach der Extraktion wurde der Druck auf 60 bar reduziert zwecks Abscheidung des Extraktes. Der Extrakt wurde der Apparatur entnommen und durch Erhitzen auf ca. 60°C vom mitextrahierten Wasser abgetrenrn. Es resultierten 12,3 g Trockenextrakt mit einem Gehalt an Hyperforin von 43,1 %.

### Beispiel 5

### Prüffing der Hyperforin-Stabilftät

In diesem Beispiel wurde der Hyperforingehalt (bestimmt mittels HPLC) eines Extraktes gemäß Beispiel 1 ohne besondere Vorsichtsmaßnahmen und Zusätze bei der Herstellung mit erfindungsgemäß hergestellten Extrakten aus den Beispielen 2-5 verglichen. Die erfindungsgemäß hergestellten Extrakte wurden unter Stickstoff und Ausschluß von Licht bei Raumtemperatur gelagert. Die Ergebnisse sind in Tabelle I zusammengefaßt. Das Ergebnis zeigt bei den erfindungsgemäß hergestellten Extrakten einen nach 12 Monaten unveränderten Hyperforingehalt. Der Gehalt an Gesamthypericin in den gemäß Beispielen 1 bis 3 hergestellten Extrakten hat sich über den gleichen Zeitraum ebenfalls nicht verändert.

**Tabelle I**

| Trockenextrakt | Hyperforin | Hyperforingehalt % | | |
|---|---|---|---|---|
| Beispiel | Ausgangsgehalt % | nach 13 Wochen | nach 6 Monaten | nach 12 Monaten |
| Beispiel 1 a) | 2,26 | 0,0 | 0,0 | 0,0 |
| Beispiel 1 b) | 2,96 | 0,0 | 0,0 | 0,0 |
| Beispiel 2 | 3,2 | 3,2 | 3,17 | 3,15 |
| Beispiel 3 | 37,2 | 37,2 | 36,5 | 36,1 |
| Beispiel 4 | 43,1 | 43,1 | 43,0 | 42,4 |

### Beispiel 6

### Weichgelatinekapseln mit Hypericumextrakt

### - Zusammensetzung

| | |
|---|---|
| Hypericum-Trockenextrakt | 300 mg |
| Ascorbinsäure | 0,25 mg |
| Octyldodecanol | 200 mg |

### Herstellung:

Als Trockenextrakt wurde der Extrakt von Beispiel 3 verwendet. Trockenextrakt und Ascorbinsäure wurden zusammen in Octyldodecanol dispergiert und unter Ausschluß von Luftsauerstoff zu Weichgelatinekapseln verarbeitet.

### Beispiel 7

### Filmtablette mit Hypericumextrakt

### - Zusammensetzung

| | |
|---|---|
| Hypericum-Trockenextrakt | 300 mg |
| Cellulose | 100 mg |
| modifizierte Stärke | 90 mg |
| Na-Carboxymethylcellulose | 30 mg |
| hochdisperses Silicumdioxid | 5,0 mg |
| Ascorbinsäure | 5,0 mg |
| Magnesiumstearat | 5,0 mg |
| Hydrorypropylmethylcellulose-Überzug | 20,0 mg |

### Herstellung:

Es wurde Trockenextrakt verwendet.

Die Bestandteile wurden in einem Mischer trocken gemischt und direkt zu Tabletten verpreßt. Die erhaltenen Tabletten wurden mit einem Überzug aus Hydroxypropylmethylcellulose beschichtet.

## Patentansprüche

1. Stabiler hyperforinhaltiger Extrakt aus Hypericum perforatum L. (Johanniskraut) mit einem Hyperforingehalt von mindestens 2%, bezogen auf den Trockenextrakt, **dadurch gekennzeichnet, dass** das Hyperforin durch einen Stabilisator aus der Gruppe bestehend aus organischen Thiolverbindungen und Ascorbinsäure und deren Derivaten in einer zur Stabilisierung des Hyperforins ausreichenden Menge gegen Zerfall oder Abbau stabilisiert ist, wobei der Stabilisator in einer Konzentration von 0,2%, bis 5%, bezogen auf den Extrakt, vorliegt.

2. Stabiler Extrakt nach Anspruch 1, **dadurch gekennzeichnet, dass** der Stabilisator in einer Konzentration von 0,2% bis 1%, bezogen auf den Extrakt, vorliegt.

3. Stabiler Extrakt nach Anspruch 1, **dadurch gekennzeichnet, dass** der Stabilisator Cystein ist.

4. Stabiler Extrakt nach Anspruch 1, **dadurch gekennzeichnet, dass** der Stabilisator Glutathion ist.

5. Stabiler Extrakt nach Anspruch 1, **dadurch gekennzeichnet, dass** der Stabilisator Ascorbinsäure ist.

6. Stabiler Extrakt nach Anspruch 1, **dadurch gekennzeichnet, dass** der Stabilisator ein Fettsäureester der Ascorbinsäure ist.

7. Verfahren zur Herstellung eines stabilen hyperforinhaltigen Extraktes, bei dem eine frische oder getrocknete Johanniskraut-Droge (Hypericum perforatum L.) mit einem pharmazeutisch üblichen anorganischen oder organischen Lösungsmittel oder dessen Gemischen, ausgenommen ölige Extraktionsmittel, extrahiert wird und bei dem ein Stabilisator aus der Gruppe bestehend aus organischen Thiolverbindungen, Ascorbinsäure und deren Derivate wahlweise während oder nach der Herstellung des Extraktes in einer Konzentration von 0,2% bis 5%, bezogen auf den Extrakt, zugesetzt wird und aus dem so erhaltenen flüssigen ein Trockenextrakt gewonnen wird.

8. Verfahren nach Anspruch 7, **dadurch gekennzeichnet, dass** als Stabilisator Cystein verwendet wird.

9. Verfahren nach Anspruch 7, **dadurch gekennzeichnet, dass** als Stabilisator Glutathion verwendet wird.

10. Verfahren nach Anspruch 7, **dadurch gekennzeichnet, dass** als Stabilisator Ascorbinsäure verwendet wird.

11. Verfahren nach Anspruch 7, **dadurch gekennzeichnet, dass** als Stabilisator ein Fettsäureester der Ascorbinsäure verwendet wird.

12. Verfahren nach Anspruch 7, **dadurch gekennzeichnet, dass** der Stabilisator in einer Konzentration von 0,2% bis 1%, bezogen auf den Extrakt, zugesetzt wird.

13. Verfahren nach Anspruch 7, **dadurch gekennzeichnet, dass** mit einem Lösungsmittel extrahiert wird, dessen Gehalt an freiem Sauerstoff niedrig ist oder stark vermindert worden ist.

14. Verfahren nach Anspruch 7, **dadurch gekennzeichnet, dass** zur Extraktion ein Lösungsmittel aus der Gruppe bestehend aus wässrigem Ethanol, wässrigem Methanol, einem Alkan mit etwa 5 bis 8 C-Atomen und flüssigem oder überkritischem Kohlendioxid verwendet wird.

15. Verfahren nach Anspruch 7, **dadurch gekennzeichnet, dass** der Stabilisator nach der Trocknung der Extraktionslösung zugesetzt wird.

16. Verfahren nach Anspruch 7, **dadurch gekennzeichnet, dass** der Stabilisator erst auf der Stufe der fertigen Arzneiform zusammen mit üblichen pharmazeutischen Hilfsstoffen zugesetzt wird.

17. Verfahren nach Anspruch 7, **dadurch gekennzeichnet, dass** das Verfahren unter Licht- und/oder Sauerstoffausschluss durchgeführt wird.

18. Pharmazeutische Zubereitung, enthaltend einen Extrakt nach einem der Ansprüche 1 bis 6 und übliche pharmazeutische Hilfsstoffe zur Behandlung von Depressionen und psychovegetativen Erkrankungen.

## Claims

1. Stable extract of Hypericum perforatum L. (St. John's wort) containing Hyperforin and having a Hyperforin content of at least 2%, based on the dry extract, **characterized in that** the Hyperforin is stabilized against decomposition or degradation by means of a stabilizer selected from the group consisting of organic thiol compounds and ascorbic acid and derivatives thereof in an amount sufficient for stabilizing the Hyperforin, wherein the stabilizer is present in a concentration of 0.2% to 5%, based on the extract.

2. Stable extract according to claim 1, **characterized in that** the stabilizer is present in a concentration of 0.2% to 1%, based on the extract.

3. Stable extract according to claim 1, **characterized in that** the stabilizer is cysteine.

4. Stable extract according to claim 1, **characterized in that** the stabilizer is glutathione.

5. Stable extract according to claim 1, **characterized in that** the stabilizer is ascorbic acid.

6. Stable extract according to claim 1, **characterized in that** the stabilizer is a fatty acid ester of ascorbic acid.

7. Process for the preparation of a stable Hyperforin-containing extract, wherein a fresh or dried St. John's wort (Hypericum perforatum L.) drug is extracted with a pharmaceutically conventional inorganic or organic solvent or mixtures thereof, with the proviso that the solvent is no oily extraction agent and wherein a stabilizer selected from the group of organic thiol compounds, ascorbic acid and derivatives thereof is added, optionally during or after the preparation of the extract in a concentration of 0.2 to 5%, based on the extract, and wherein a dry extract is obtained from the thus obtained liquid extract.

8. Process according to claim 7, **characterized in that** cysteine is used as stabilizer.

9. Process according to claim 7, **characterized in that** glutathione is used as stabilizer.

10. Process according to claim 7, **characterized in that** ascorbic acid is used as stabilizer.

11. Process according to claim 7, **characterized in that** a fatty acid ester of ascorbic acid is used as stabilizer.

12. Process according to claim 7, **characterized in that** the stabilizer is added in a concentration of 0.2% to 1%, based on the extract.

13. Process according to claim 7, **characterized in that** a solvent is used for extraction, the free oxygen content of which is low or was considerably reduced.

14. Process according to claim 7, **characterized in that** a solvent is used for extraction selected from the group of aqueous ethanol, aqueous methanol, alkane having about 5 to 8 C-atoms, and liquid or supercritical carbon dioxide.

15. Process according to claim 7, **characterized in that** the stabilizer is added after drying the extract solution.

16. Process according to claim 7, **characterized in that** the stabilizer is added only at the stage of the finished pharmaceutical composition together with conventional pharmaceutical additives.

17. Process according to claim 7, **characterized in that** the process is carried out by excluding light and/or oxygen.

18. Pharmaceutical composition containing an extract according to any of claims 1 to 6 and conventional pharmaceutical additives for the treatment of depressions and psychovegetative disorders.

## Revendications

1. Extrait stable de Hypericum perforatum L. (herbe de la Saint-Jean) contenant de l'hyperforine, qui a une teneur en hyperforine d'au moins 2 %, sur la base de l'extrait sec, **caractérisé en ce que** l'hyperforine est stabilisée vis-à-vis de la décomposition ou de la dégradation au moyen d'un stabilisant choisi dans le groupe des composés de type thiol organiques et de l'acide ascorbique et de ses dérivés en une quantité suffisante, le stabilisant étant présent dans une concentration de 0,2 % à 5 %, sur la base de l'extrait.

2. Extrait stable selon la revendication 1, **caractérisé en ce que** le stabilisant est présent dans une concentration de 0,2 % à 1 %, sur la base de l'extrait.

3. Extrait stable selon la revendication 1, **caractérisé en ce que** le stabilisant est de la cystéine.

4. Extrait stable selon la revendication 1, **caractérisé en ce que** le stabilisant est du glutathion.

5. Extrait stable selon la revendication 1, **caractérisé en ce que** le stabilisant est de l'acide ascorbique.

6. Extrait stable selon la revendication 1, **caractérisé en ce que** le stabilisant est un ester d'acide gras de l'acide ascorbique.

7. Procédé pour la préparation d'un extrait stable contenant de l'hyperforine, dans lequel une substance médicamenteuse à base d'herbe brute fraîche ou séchée (Hypericum perforatum L.) est extraite avec un solvant inorganique ou organique pharmaceutique conventionnel ou des mélanges de celui-ci, à condition que les solvants ne soient pas des agents d'extraction huileux, et dans lequel un stabilisant choisi dans le groupe des composés de type thiol organiques, de l'acide ascorbique et de ses dérivés est ajouté, en option, pendant ou après la préparation de l'extrait, dans une concentration de 0,2 % à 5 %, sur la base de l'extrait, et dans lequel un extrait sec est obtenu à partir de l'extrait liquide ainsi obtenu.

8. Procédé selon la revendication 7, **caractérisé en ce que** de la cystéine est utilisée comme stabilisant.

9. Procédé selon la revendication 7, **caractérisé en ce que** du glutathion est utilisé comme stabilisant.

10. Procédé selon la revendication 7, **caractérisé en ce que** de l'acide ascorbique est utilisé comme stabilisant.

11. Procédé selon la revendication 7, **caractérisé en ce qu'**un ester d'acide gras de l'acide ascorbique est utilisé comme stabilisant.

12. Procédé selon la revendication 7, **caractérisé en ce que** le stabilisant est ajouté dans une concentration de 0,2 % à 1 %, par rapport à l'extrait.

13. Procédé selon la revendication 7, **caractérisé en ce qu'**on utilise pour l'extraction un solvant dont la teneur en oxygène libre est faible ou a été considérablement réduite.

14. Procédé selon la revendication 7, **caractérisé en ce qu'**on utilise pour l'extraction un solvant choisi dans le groupe de l'éthanol aqueux, du méthanol aqueux, d'un alcane ayant environ 5 à 8 atomes de carbone, et du dioxyde de carbone liquide ou supercritique.

15. Procédé selon la revendication 7, **caractérisé en ce que** le stabilisant est ajouté après séchage de la solution d'extrait.

16. Procédé selon la revendication 7, **caractérisé en ce que** le stabilisant est ajouté seulement au stade de la composition pharmaceutique finie conjointement avec des additifs pharmaceutiques conventionnels.

17. Procédé selon la revendication 7, **caractérisé en ce que** le procédé est mis en oeuvre à l'abri de la lumière et/ou de l'oxygène.

18. Composition pharmaceutique contenant un extrait selon l'une quelconque des revendications 1 à 6 et des additifs pharmaceutiques conventionnels pour le traitement des dépressions et des troubles psychovégétatifs.
